Europäisches Patentamt

European Patent Office

Office européen des brevets

(11) Publication number: **0 212 929 B1**

# EUROPEAN PATENT SPECIFICATION

(45) Date of publication of patent specification: **22.05.91**

(51) Int. Cl.⁵: **A61F 2/30**, A61C 8/00, A61L 27/00

(21) Application number: **86306173.5**

(22) Date of filing: **08.08.86**

(54) Method for producing endosseous implants.

(30) Priority: **08.08.85 JP 175568/85**
**08.08.85 JP 175570/85**

(43) Date of publication of application:
**04.03.87 Bulletin 87/10**

(45) Publication of the grant of the patent:
**22.05.91 Bulletin 91/21**

(84) Designated Contracting States:
**CH DE FR GB IT LI SE**

(56) References cited:
**DE-A- 2 313 678**
**FR-A- 2 318 617**
**FR-A- 2 336 913**
**FR-A- 2 374 019**
**FR-A- 2 374 020**

(73) Proprietor: **SUMITOMO CHEMICAL COMPANY, LIMITED**
**Kitahama 4-chome 5-33**
**Chuo-ku Osaka 541(JP)**

(72) Inventor: **Hama, Masaaki**
**3810 NE, 75th St. Apt. No. 1**
**Seattle Washington 98115(US)**
Inventor: **Watanabe, Keiichirou**
**2-11-7-508, Sone-Higashi-cho**
**Toyonaka-shi Osaka-fu(JP)**
Inventor: **Ishimaru, Hiroshi**
**2-3, Ikku-cho**
**Niihama-shi Ehime-ken(JP)**

(74) Representative: **Bizley, Richard Edward et al**
**BOULT, WADE & TENNANT 27 Furnival Street**
**London EC4A 1PQ(GB)**

## Description

This invention relates to a method for producing endosseous implants.

Implantation technology, which comprises the insertion of artificial materials such as artificial organs, artificial blood vessels, artificial joints, artificial bones and artificial tooth roots into the body, has attracted much attention in recent years. It is said that trials of implantation go back to ancient times. Particularly in the last ten years, a huge number of treatments by implantation have been performed on bones and tooth roots to afford good results in the remedy of defects or the recovery of functions thereof. However, there has not yet been obtained an artificial bone or tooth root which satisfies the necessary requirements as a material for living bodies, i.e. affinity to living tissue, safety and excellent durability.

As metallic materials which have mainly been used for preparation of artificial bones or tooth roots, cobalt-chromium alloys, stainless steel, titanium and tantalum are exemplified. On the other hand, as ceramic materials, alumina or materials comprising predominantly carbon have been recently taken note of.

Although metallic materials are excellent in mechanical strength, particularly in impact strength, they lack affinity for living tissue. For example, when a metallic implant is used, metal ions are dissolved out therefrom and are toxic to bone cells around the implant. Furthermore, bone-formation is obstructed, probably because of too large a thermal conductivity of the metallic implant. Among the metallic materials, titanium and tantalum are particularly superior in a corrosion-resistance and hence have been employed as fixing plates for skulls or fractured parts of bones and implants of jawbones since about 1940, but these are not necessarily satisfactory.

On the other hand, ceramic materials generally show a good affinity to bones, and tissues penetrate into fine pores of ceramic materials to afford a strong fixation, without adverse reaction between the ceramic material and the tissue. Besides, they are also excellent in durability, that is, they are resistant to corrosion or decomposition. On the other hand, they possess a poor impact strength.

There has been proposed an implant having the characteristics of both metallic materials and ceramic materials, i.e. an implant prepared by thermally spraying a ceramic material onto the surface of a metallic core material (cf. Japanese Patent First Publication Nos. 14095/1977, 82893/1977, 28997/1978 and 75209/1978). In these methods, however, a self-bonding type bonding agent is used in order to improve the adhesion of the ceramic coating layer. The bonding agent has the problem that it contains nickel, chromium, etc. which dissolve out in living tissue and have toxic effects on the body.

FR-A-2336913 describes an implant made from a metallic core material, the surface of which is subjected to sand-blasting before spraying with a ceramic material.

The present invention provides endosseous implants prepared by thermally spraying a ceramic material onto a metallic core material having a rough surface. More particularly, there is provided a method for producing an endosseous implant by thermally spraying a ceramic material onto a metallic core material having a maximum surface roughness of 15 to 100 $\mu$m.

In accordance with the invention there is provided a method for producing an endosseous implant which comprises the steps of:

(a) roughening the surface of a metallic core material and

(b) thermally spraying a ceramic material onto the roughened surface of the metallic core material;

characterised in that the surface of said metallic core material is roughened to a maximum surface roughness of 15 to 100 $\mu$m by thermally spraying titanium hydride thereon.

The present invention will now be further described by way of example only with reference to the accompanying drawings, in which:

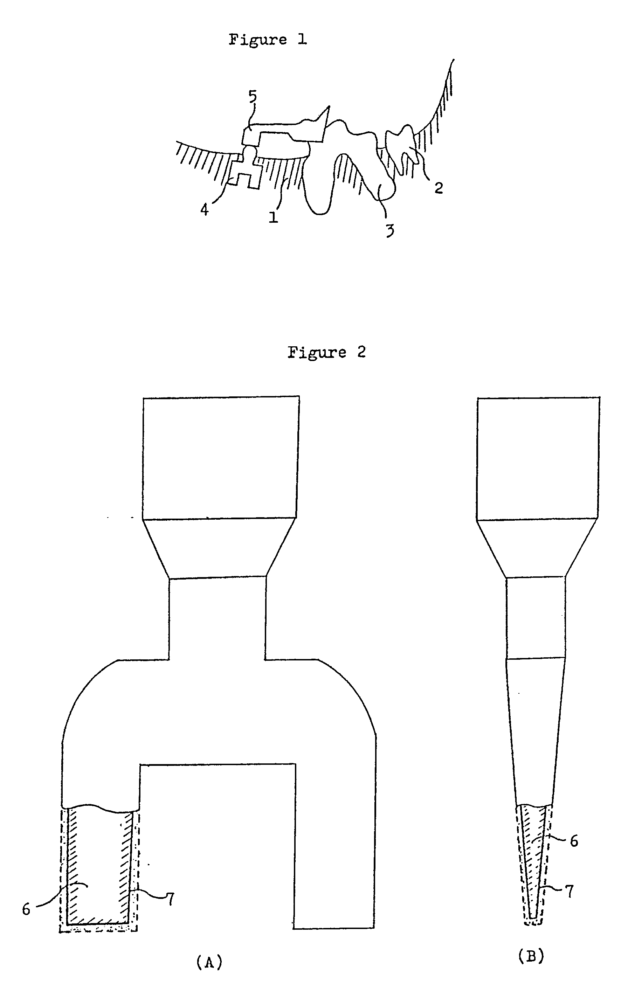

Figure 1 is a schematic view of an embodiment of the endosseous implant for the lower jawbone of a dog, wherein 1 represents the lower jawbone, 2 and 3 are natural teeth, 4 is an artificial tooth root and 5 is an artificial tooth crown attached on the artificial tooth root 4.

Figure 2 is a schematic view of an embodiment of the endosseous implant of blade type for the jawbone according to this invention, and (A) is a front view thereof and (B) is a side view thereof, wherein 6 represents a metallic implant (core material) and 7 is a ceramic layer containing unopened pores which do not reach the metal surface.

According to the present invention, as is shown in Figure 2, a ceramic coating is applied to the surface of a metallic implant core material so as to obtain an implant with good impact strength and interacting with the surrounding bone tissues in a similar manner to ceramic materials.

The metallic core materials used in this invention may be any suitable materials which have appropriate mechanical strength and are not harmful to the body, including those materials which have usually been used as artificial materials for bones, joints and tooth roots which do not exhibit harmful effects on living tissue and possess an appropriate mechanical strength, for example, cobalt-chromium alloys, stainless steels, titanium, titanium alloys, tantalum or zirconium. Among these materials, preferred are titanium, titanium alloys, zirconium and tantalum in view of their excellent corrosion resistance. Most preferred are titanium and titanium alloys (e.g. 6 % A1-4 % V-Ti, etc.) in view of their excellent processability and safety.

The ceramic materials used in this invention may be, for example, hydroxyapatite, calcium phosphate, aluminum oxide, zirconium oxide or titanium oxide, which may be used alone or in combination of two or more thereof. In order to control the pores in the ceramic layer, porcelain may be applied by thermally spraying it together with the ceramic material or by baking it on the ceramic coating layer. For such a purpose, there can be used porcelains such as Dentin and Enamel, for example. Among the ceramic materials, preferred are hydroxyapatite and aluminum oxide in view of their excellent affinity with living tissue. A combination of hydroxyapatite and aluminum oxide is particularly suitable because it interacts most intimately with living tissue.

The endosseous implants of this invention can be produced in the following manner.

The metallic material is formed into the desired shape, for example by conventional methods, such as cutting, casting, forging, punching, electroarc machining, laser-processing, or powdered metal technique. The surface of the metallic core material thus formed is roughened to a specific maximum surface roughness. The maximum surface roughness of the metallic core materials is in the range of 15 $\mu$m to 100 $\mu$m, the inventors having measured the roughness by the method described in JIS B-0601. The expression "maximum surface roughness" means "peak to valley height Rt" as defined in "Encyclopedia of Material Science and Engineering" (PERGAMON PRESS) volume 6, page 4806, right column and Figure 1. When the maximum surface roughness is smaller than 15 $\mu$m, the thermally sprayed ceramic coating layer shows insufficient adhesion. On the other hand, when it is larger than 100 $\mu$m, it is disadvantageously difficult to form a thin uniform layer of the ceramic coating. The most suitable maximum surface roughness is in the range of 20 to 60 $\mu$m in view of the adhesion and uniformity of the coating layer.

In order to roughen the surface of the metallic core materials, various methods may be applied, for example, mechanical methods such as grinding, sandblasting, grit blasting, etc.; chemical etching, e.g. treatment with an acid or alkali; electrolytic etching before forming a titanium layer with a rough surface by thermally spraying titanium hydride powder. Forming a titanium layer with a rough surface allows the ceramic material to easily bite into the rough surface.

Chemical etching is usually carried out by using mineral acids, for example sulfuric acid, hydrochloric acid or hydrofluoric acid, which may be used alone or in a combination of two or more thereof. When the metallic core materials are coated with titanium having a rough surface by thermally spraying titanium hydride powder, it is preferable to previously subject the materials to the above surface-roughening treatments, for example mechanical treatment (e.g. grinding, sandblasting, grit blasting), chemical etching with an acid or alkali or electrolytic etching. The thermal spraying of titanium hydride is preferably carried out by thermal plasma spraying. The particle size of the titanium hydride is not particularly limited, but is preferably in the range of 10 to 100 $\mu$m. The titanium coating layer substantially does not release any harmful metal ion, unlike the self-bonding type bonding agent-containing metals which are easily dissolved out in living tissue.

In the thermal spraying of ceramic materials, any portion which is not coated with the ceramic material is previously masked by an appropriate means, for instance, application of a marking ink or an aluminum adhesive tape prior to the treatment for roughening the surface. The thermal spraying of the ceramic material is also preferably carried out by a thermal plasma spraying apparatus. Some portions of the endosseous implants, for instance, the ceramic coating layer in artificial joints, are required to be highly smooth. In such a case, a porcelain is coated onto the surface and the coated product is repeatedly calcined in a vacuum furnace.

In the endosseous implants of this invention, the thickness of the ceramic coating layer which optionally contains porcelain is not particularly limited, but is preferably in the range of 10 to 200 $\mu$m.

This invention is illustrated by the following Examples but should not be construed to be limited thereto. In the Examples, the surface roughness figures are as measured by the method of JIS B-0601.

Example 1

A core material for an endosseous implant is prepared from a titanium material (JIS, second class of material) by cutting and grinding the titanium material by electro arc machining.

The metallic core material for the implant is grit-blasted with a blast apparatus [a mammoth type ventiblast apparatus, manufactured by Metco Inc., England; blasting agent: Metcolite VF, manufactured by Metco Inc.; pressure: 30 psi (205 kPa)]. The thus-blasted material has a maximum surface roughness of 10 $\mu$m.

Under an argon-hydrogen-plasma jet flame (ARC electric current 500 Amp) generated by a plasma spray apparatus (6MM-630 type, manufactured by Metco Inc., equipped with an electric power supplier), titanium hydride powder (Powder No. XP-1157, manufactured by Metco Inc.) is thermally sprayed, as the first coating layer, onto the blasted core material to form a first coating layer of about 50 $\mu$m in thickness over the whole surface thereof. As the second coating layer, a mixture of hydroxyapatite (particle size 10 - 100 $\mu$m, 80 % by weight) and aluminum oxide (WA #120, manufactured by Nippon Kenmazai K.K. 20 % by weight) is thermally sprayed to form a coating layer having a thickness of about 150 $\mu$m on average. The thermally sprayed coating layer has excellent adhesion, and even when the product is subjected to a bending process at an angle of 160°, the coating layer is not peeled off.

The product obtained above was tested as follows:

The implant was embedded into the lower jawbone of a dog. After 3 months, it was observed by X-ray fluoroscopy. As a result, there was confirmed formation of dense bone around the implant.

Reference Example

A core material for an endosseous implant is prepared by treating the same titanium material in the same manner as described in Example 1. The core material is likewise subjected to grit blasting, but is not subjected to etching. The material has a maximum surface roughness of 10 $\mu$m which is about 1/5 of that of the core material before thermal spraying in Example 1.

The blasted core material is thermally sprayed with a mixture of hydroxyapatite and aluminum oxide in the same manner as in Example 1 to give a coating layer having a thickness of about 150 $\mu$m on average. The resulting product has significantly inferior adhesion of the coating layer and the coating layer is easily peeled off even by a light impact. Thus, this product cannot be used as an endosseous implant.

It will be appreciated from the foregoing that in accordance with the invention there may be provided endosseous implants comprising a metallic core whose surface is roughened to a maximum roughness of 15 to 100 $\mu$m and a ceramic material directly bonded to the metallic core without a bonding agent. Roughening is provided by a titanium layer on the metallic core and, optionally, the surface of the metallic core below the titanium layer is roughened. This roughening of the surface of the metallic core which acts as a substrate for the titanium layer may be achieved by blasting or chemical etching or both.

It will also be seen from the foregoing that we have provided an endosseous implant which has good impact strength and the affinity for living tissue of ceramics but need not release toxic metal ions into the body.

## Claims

1. A method for producing an endosseous implant which comprises the steps of:-
   (a) roughening the surface of a metallic core material (6) and
   (b) thermally spraying a ceramic material (7) onto the roughened surface of the metallic core material;
   characterised in that the surface of said metallic core material is roughened to a maximum surface roughness of 15 to 100 $\mu$m by thermally spraying titanium hydride thereon.

2. A method according to claim 1 characterised in that the surface of the metallic core material (6) is subjected to blasting or etching with an acid or both before being thermally sprayed with titanium hydride.

3. A method according to claim 1 or claim 2 characterised in that the titanium hydride has a particle size of 10 to 100 $\mu$m.

4. A method according to any one of the preceding claims, characterised in that the metallic core material (6) has a maximum surface roughness of 20 to 60 $\mu$m.

5. A method according to any one of the preceding claims, characterised in that the ceramic material (7) for thermal spray coating comprises hydroxyapatite.

6. A method according to any one of claims 1 to 4, wherein the ceramic material (7) for thermal spray coating comprises a mixture of hydroxyapatite and aluminum oxide.

7. A method according to either of claims 5 or 6 characterised in that thermal spraying of the ceramic material results in a layer (7) of 10 to 200 $\mu$m in thickness.

8. A method according to any one of the preceding claims characterised in that the metallic core material is selected from cobalt-chromium alloys, stainless steel, titanium, titanium alloys, tantalum and zirconium.

9. A method according to claim 8 characterised in that the metallic core material is titanium or a titanium alloy.

**Revendications**

1. Procédé de production d'un implant intra-osseux comprenant les étapes consistant à :
   (a) rendre rugueuse la surface du matériau d'un noyau métallique (6) et
   (b) pulvériser à chaud un matériau céramique (7) sur la surface rugueuse du matériau du noyau métallique ;
   caractérisé en ce que la surface dudit matériau du noyau métallique est rendue rugueuse jusqu'à une rugosité superficielle maximale comprise entre 15 et 100 $\mu$m, par pulvérisation à chaud d'· hydrure de titane.

2. Procédé selon la revendication 1, caractérisé en ce que la surface du matériau (6) du noyau métallique est soumise à un décapage mécanique ou à un décapage chimique par un acide ou à ces deux procédés avant la pulvérisation à chaud de l'hydrure de titane.

3. Procédé selon la revendication 1 ou la revendication 2, caractérisé en ce que l'hydrure de titane a une granulométrie comprise entre 10 et 100 $\mu$m.

4. Procédé selon l'une quelconque des revendications précédentes, caractérisé en ce que le matériau (6) du noyau métallique présente une rugosité superficielle maximale comprise entre 20 et 60 $\mu$m.

5. Procédé selon l'une quelconque des revendications précédentes, caractérisé en ce que le matériau céramique (7) destiné à être pulvérisé à chaud est constitué par de l'hydroxyapatite.

6. Procédé selon l'une quelconque des revendications 1 à 4, dans lequel le matériau céramique (7) destiné à être pulvérisé à chaud est constitué par un mélange d'hydroxyapatite et d'oxyde

7. Procédé selon la revendication 5 ou la revendication 6, caractérisé en ce que la pulvérisation à chaud du matériau céramique donne une couche (7) de 10 à 200 $\mu$m d'épaisseur.

8. Procédé selon l'une quelconque des revendications précédentes, caractérisé en ce que le matériau du noyau métallique est sélectionné parmi des alliages au cobalt-chrome, de l'acier inoxydable, du titane, des alliages de titane, du tantale et du zirconium.

9. Procédé selon la revendication 8, caractérisé en ce que le matériau du noyau métallique est du titane ou un alliage de titane.

**Ansprüche**

1. Verfahren zum Herstellen eines Implantats für den Einsatz in einem Knochen, mit folgenden Schritten:
   (a) Anrauhen der Oberfläche eines metallischen Kernmaterials (6) und
   (b) thermisches Aufsprühen eines keramischen Materials (7) auf die angerauhte Oberfläche des metallischen Kernmaterials, **dadurch gekennzeichnet**, daß die Oberfläche des metallischen Kernmaterials angerauht wird auf eine maximale Oberflächenrauhigkeit von 15 bis 100 $\mu$m durch thermisches Aufsprühen von Titanhydrid.

2. Verfahren nach Anspruch 1, **dadurch gekennzeichnet**, daß die Oberfläche des metallischen Kernmaterials (6) bevor das Titanhydrid thermisch aufgesprüht wird gestrahlt und/oder mit einer Säure geätzt wird.

3. Verfahren nach Anspruch 1 oder 2, **dadurch gekennzeichnet**, daß das Titanhydrid eine Teilchengröße von 10 bis 100 $\mu$m aufweist.

4. Verfahren nach einem der vorstehenden Ansprüche, **dadurch gekennzeichnet**, daß das metallische Kernmaterial (6) eine maximale Oberflächenrauhigkeit von 20 bis 60 $\mu$m aufweist.

5. Verfahren nach einem der vorstehenden Ansprüche, **dadurch gekennzeichnet**, daß das keramische Material (7) zur thermischen Aufsprühbeschichtung Hydroxyapatit aufweist.

6. Verfahren nach einem der Ansprüche 1 bis 4, wobei das keramische Material (7) zur thermischen Aufsprühbeschichtung eine Mischung aus Hydroxyapatit und Aluminiumoxid aufweist.

7. Verfahren nach Anspruch 5 oder 6, **dadurch gekennzeichnet**, daß das thermische Aufsprühen des keramischen Materials zu einer Schicht (7) mit einer Dicke von 10 bis 200 $\mu$m führt.

8. Verfahren nach einem der vorstehenden Ansprüche, **dadurch gekennzeichnet**, daß das metallische Kernmaterial ausgewählt wird aus Kobalt-Chromlegierungen, rostfreiem Stahl, Titan, Titanlegierungen, Tantal und Zirkon.

9. Verfahren nach Anspruch 8, **dadurch gekennzeichnet**, daß das metallische Kernmaterial Titan oder eine Titanlegierung ist.

Figure 1

Figure 2

(A)

(B)